Europäisches Patentamt

European Patent Office

Office européen des brevets

(19)

(11) Publication number: **0 165 669**

**A1**

(12) # EUROPEAN PATENT APPLICATION

(21) Application number: **85302538.5**

(22) Date of filing: **11.04.85**

(51) Int. Cl.⁴: **G 01 N 33/53**
**G 01 N 33/543, G 01 N 33/546**
**G 01 N 33/551, G 01 N 33/78**

(30) Priority: **04.05.84 US 607148**

(43) Date of publication of application:
**27.12.85 Bulletin 85/52**

(84) Designated Contracting States:
**DE FR GB IT**

(71) Applicant: **Corning Glass Works**
**Houghton Park**
**Corning New York 14831(US)**

(72) Inventor: **Buehler, Robert J.**
**12 Russell Circle**
**Natick, MA 01760(US)**

(72) Inventor: **Riceberg, Louis J.**
**Daikanyama Tower No. 206 1-35-11 Ebisunishi**
**Shibuya-Ku Tokyo 150(JP)**

(72) Inventor: **Odstrchel, Gerald**
**20 Country Club Dr.**
**Walpole, MA 02081(US)**

(74) Representative: **Smith, Sydney et al,**
**Elkington and Fife High Holborn House 52/54 High**
**Holborn**
**London WC1V 6SH(GB)**

(54) **Improved method of measuring free ligand.**

(57)    A single step free ligand immunoassay based on the use of a labeled ligand or label ligand derivative which is substantially nonreactive with the natural binders present in the sample to be analysed is improved through the inclusion in the assay of an amount of a blocking agent which is sufficient to prevent significant binding of the labeled ligand or labeled ligand derivative to various binding agents without causing significant release of bound ligand.

EP 0 165 669 A1

Croydon Printing Company Ltd.

- 1 -

# IMPROVED METHOD OF MEASURING FREE LIGAND

The present invention relates to the measurement of free ligand in a liquid sample which contains the ligand in both free and bound states. More particularly, the present invention relates to an improvement in the measurement of free ligand in a liquid sample which contains the ligand in both free and combined states.

Various species are known to be present in biological fluids in both free and combined states. In the combined state, each species typically is bound to one or more endogenous proteins, i.e., proteins which are normally present in the biological fluids. There often is a single protein which is primarily responsible for such binding. Moreover, the amount of a given species which is present in the free state usually represents only a very small percentage of the total amount of the species present in the biological fluid and typically is more significant clinically than the total amount of the species or the amount of the species which is present in the bound state. There are times, however, when it is either desirable or necessary to know both the amount of the species which is present in the free state and the total amount of the species present in the biological fluid.

Perhaps the most thoroughly studied examples of species fitting the foregoing description are the thyroid hormones, e.g., thyroxine and 3,5,3'-triiodothyronine. Approximately 99.97% of the thyroxine and about 99.7% of the 3,5,3'-triiodothyronine in human serum are bound to endogenous serum proteins, especially thyroxine binding globulin (TBG).

For convenience, the discussion which follows is directed to thyroid hormones. Such discussion, however, is not to be construed as in any manner limiting either the spirit or scope of the present invention.

The classical method for determining the amount of free thyroxine or 3,5,3'-triiodothyronine in human serum, typically expressed as a concentration in ng/dl, is equilibrium dialysis. While accurate, the method is very time consuming and not suitable for routine testing in a clinical laboratory, hospital, or similar setting.

Not surprisingly, then, prior art efforts have been directed to overcoming the disadvantages inherent with equilibrium dialysis. One such effort is represented by U.S. Patent No. 4,046,870 which discloses a method for determining the concentration of free thyroid hormone in a blood serum sample, which method comprises the steps of analyzing the sample via immunoassay techniques for either thyroid hormone in the presence and absence of a blocking agent to establish a thyroid hormone binding differential and then correlating that differential with a standard curve relating the respective free thyroid hormone concentrations to binding differentials. The blocking agent typically was thiomersal at a concentration of 2.5 mg/ml.

It will be recognized by those having ordinary skill in the art that the immunoassay carried out in the presence of a blocking agent gives a result which can be correlated with the total thyroid hormone concentration through the use of an appropriate dose-response or standard curve. The procedure, then, is especially advantageous when it is necessary or desirable to know both free and total thyroid hormone concentrations. When only the free thyroid hormone concentration is desired, however, the procedure still requires two separate measurements. Consequently, such procedure often has been deemed less desirable than one in which a single measurement is required for the determination of free thyroid hormone.

An example of such a single-measurement assay (a so-called single step free ligand immunoassay) is represented by U.S. Patent No. 4,366,143 to Midgley and Wilkins. The disclosed method involves the use of a labeled ligand derivative which is substantially nonreactive with the natural binders present in the biological fluid sample to be analyzed.

The concept of substantial nonreactivity of the labeled ligand with endogenous binding proteins is the basis of at least three commercially available kits for measuring free thyroid hormone in human serum. See, in addition, U.S. Patent No. 4,410,633 which also describes the concept.

In practice, all of the presumably substantially nonreactive labeled thyroxine derivatives do, in fact, bind to at least some of the endogenous serum proteins. Moreover, many of the commercially available kits require the addition of bovine serum albumin to the reaction media, which albumin can influence the free thyroxine values, presumably because the labeled thyroxine derivatives bind at least in part to the added albumin. See, by way of illustration, Amino et al., Clin. Chem., 29, 321 (1983); Stockigt et al. in Albertini and Ekins, Editors, "Free Hormones In Blood," Elsevier Biomedical Press, Amsterdam, 1982, p. 223; Helenius and Liewendahl, Clin. Chem., 29, 816 (1983); Byfield et al., Clin. Endroclinol., 19, 277 (1983); and Stockigt et al., Clin. Chem., 29, 1408 (1983).

The above-described, at-least-partial binding of the labeled thyroxine derivatives employed in the single step free thyroxine immunoassays would not be of concern if the serum sample compositions remained essentially constant from patient to patient. As shown in part by the above-cited references, however, such is not the case.

Abnormalities in serum protein concentrations and compositions occur which result in falsely elevated free thyroxine measurements. That is, the single step free thyroxine assays exhibit above-normal free thyroxine levels with samples from patients with thyroxine antibodies (increased IgG) and variations in serum albumin or prealbumin levels. Such above-normal free thyroxine levels result from increased binding of the labeled thyroxine derivative to the IgG, albumin, or prealbumin which in turn leads to reduced binding to the specific binder, e.g., anti-thyroxine antibody, employed in the assay, a lower count rate, and, consequently, higher-than-expected free thyroxine values.

On the other hand, lower-than-expected free thyroxine values result with patient samples which contain certain interfering compounds, such as aspirin, diphenylhydantoin, phenylbutazone, and the like. Such interfering compounds act as blocking agents (see, for example, U.S. Patent No. 3,911,096 to Chopra) which cause the release of labeled thyroxine derivative from exogenous bovine serum albumin and/or endogenous binding proteins. The released derivative then is available to bind to the specific binder employed in the assay. This results in a higher count rate and, therefore, lower thyroxine values. The clinical consequence is the misdiagnosis of normal patients receiving certain types of medication as hypothyroid or borderline hypothyroid.

## Summary of the Invention

It therefore is an object of the present invention to provide an improved method of measuring free ligand in a liquid sample which contains the ligand in both free and bound states.

It is a further object of the present invention to provide an improved method of measuring free ligand in a liquid sample which contains the ligand in both free and bound states, which method is more diagnostically specific and has fewer false positives in the hypothyroid range than some prior art methods.

Yet another object of the present invention is to provide an improved single step free ligand immunoassay.

Still another object of the present invention is to provide an improved single step free ligand immunoassay which is more diagnostically specific and has fewer false positives in the hypothyroid range than prior art single step free ligand immunoassays.

These and other objects will be readily apparent to those having ordinary skill in the art from a consideration of the specification and claims which follow.

Accordingly, the present invention provides, in a method of determining the concentration of a free ligand in a liquid sample which contains the ligand in both free and bound states, which method comprises the steps of:

A. adding to the liquid sample labeled ligand or labeled ligand derivative which does not bind significantly to the specific endogenous ligand binding protein, but does bind significantly to other proteins, and a binder specific for the ligand and labeled ligand or labeled ligand derivative;

B. incubating the mixture resulting from step A;

C. measuring the amount of labeled ligand or labeled ligand derivative which is bound to the specific binder; and

D. using the measurement to determine the concentration of the free ligand in the liquid sample; the improvement which comprises adding to the sample in step A an amount of a blocking agent which is sufficient to prevent significant binding of the labeled ligand or labeled ligand derivative to endogenous and/or exogenous ligand binding proteins without causing significant release of bound ligand.

In a preferred embodiment, the ligand is a thyroid hormone, such as thyroxine or 3,5,3'-triiodothyronine.

In another preferred embodiment, the specific binder is immobilized on a water-insoluble support. In a more preferred embodiment, the water-insoluble support is particulate and, optionally, paramagnetic.

The results obtained by the addition of a blocking agent as described herein are both surprising and unexpected in view of (1) the well-known function of blocking agents to release bound ligand from endogenous ligand binding proteins and (2) the fact that, at least in some instances, the principal effect of the blocking agent is on the specific ligand binding protein (see U.S. Patent No. 4,046,870).

### Detailed Description of the Invention

As used herein, the term "endogenous ligand binding proteins" includes those ligand binding proteins which are naturally present in a given biological sample.

The term "exogenous ligand binding proteins" includes those proteins which are added to the sample during either sample preparation or the assay procedure and which are capable of at least partially binding the labelled ligand or labelled ligand derivative. For example, many free thyroxine assay procedures require the addition of bovine serum albumin to the sample. Such bovine serum albumin typically is capable of at least partially binding the labelled thyroxine or labelled thyroxine derivative employed in the assay. Thus, such exogenous ligand binding proteins are similar to the endogenous ligand binding proteins in their affinity for labelled ligand or labelled ligand derivative.

The term "specific endogenous ligand binding protein" refers to the endogenous protein which is primarily responsible for binding ligand. For example, the specific endogenous thyroxine binding protein is thyroxine-binding globulin (TBG).

In general, the blocking agent can be any compound which serves to prevent significant binding of the labeled ligand to endogenous and/or exogenous ligand binding proteins. Note U.S. Patent No. 3,911,096, cited supra. Suitable blocking agents include, among others, 8-anilino-1-naphthalenesulfonic acid, 3-(4-anilino-1-naphthylazo)-2,7-naphthalenedisulfonic acid, 2,4,6-trinitrobenzenesulfonic acid, α-naphthalenesulfonic acid, sodium ethylmercurithiosalicylate (thiomersal), sodium salicylate, phenytoin (diphenylhydantoin or 5,5-diphenyl-2,4-imidazolidinedione), doxepin, diazapam, prochlorperazine, phenylbutazone, propylthiouracil, methimazole, and the like. Preferred blocking agents include phenytoin and sodium salicylate.

The blocking agent is employed in an amount which is sufficient to prevent significant binding of the labeled ligand or labeled ligand derivative to endogenous and/or exogenous ligand binding proteins without causing significant release of bound ligand. Such amount will vary, depending upon the blocking agent and the labeled ligand or labeled ligand derivative, among other factors. However, one having ordinary skill in the art can readily determine such amount without undue experimentation.

Clearly, such amount will be significantly less than the amount which normally would be used in a total ligand assay. For example, the amounts of blocking agents employed in U.S. 3,911,096 varied from 50 μg to 10 mg per 25 μl of serum, which amounts displaced from 3.0 to greater than 20 ng of thyroxine from TBG. Human serum normally contains no more than about 12.5 μg of thyroxine per dl, which amount corresponds to about 3.1 ng per 25 μl sample. With the possible exception of α-naphthalenesulfonic acid, all of the blocking agents were employed in amounts which released at least 3.1 ng of thyroxine from TBG.

As can be seen in the Examples, sodium salicylate was suitably employed in a free thyroxine radioimmunoassay at a concentration of 0.375 g/l in the tracer solution. Since the amount of tracer solution utilized was 200 μl and the sample size was 50 μl, the amount of sodium salicylate per 25 μl of sample would be 37.5 μg. In U.S. 3,911,096, however, sodium salicylate was tested at a level of 10 mg per 25 μl of sample, a 267-fold increase over the level employed in the Examples.

It should be apparent to those having ordinary skill in the art that the method of the present invention is useful in any single step free ligand immunoassay which utilizes a labeled ligand or labeled ligand derivative which is substantially nonreactive with binding proteins present in the sample, irrespective of the particular protocol employed.

By way of illustration only, the present invention is further described by the Examples which follow, without intending to in any way limit the spirit or scope of the invention.


## Example 1


Corning IMMO PHASE[TM] Single Step $FT_4$ [$^{125}I$] Radioimmunoassay kits (Corning Medical, Corning Glass Works, Medfield, Massachusetts) were used. The procedure recommended by the manufacturer was followed. The free thyroxine standards and thyroxine antibody reagent supplied with the kits were used without modification. For assays conducted in the absence of a blocking agent, the tracer reagent supplied with the kits also was used without modification. For assays conducted in the presence of a blocking agent, in accordance with the present invention, sodium salicylate was added to kit tracer reagent at a level of 0.375 g/1 which is equivalent to 0.375 mg/ml.

Tables I and II which follow summarize clinical data obtained with a normal population.

### Table I

### Normal Population

| Patient I.D. No. | $FT_4$ E.D.[a] | Single Step $FT_4$ Assay | |
|---|---|---|---|
| | | Without Salicylate[b] | With Salicylate[b] |
| 8657 | 1.3 | 0.04 | 0.51 |
| 8829 | – | 0.11 | 0.64 |
| 8654 | 2.5 | 0.43 | 1.15 |
| 8635 | 1.1 | 0.54 | 0.91 |
| 8630 | 0.8 | 0.59 | 0.88 |
| 8553 | 1.7 | 0.61 | 0.63 |
| 8673 | 1.0 | 0.73 | 0.46 |
| 8604 | 1.0 | 0.79 | 0.93 |
| 8565 | 1.4 | 0.80 | 1.16 |
| 8658 | 1.0 | 0.86 | 0.88 |
| 8536 | 1.9 | 0.89 | 1.20 |

| Patient I.D. No. | $FT_4$ E.D.[a] | Single Step $FT_4$ Assay | |
|---|---|---|---|
| | | Without Salicylate[b] | With Salicylate[b] |
| 8557 | 1.0 | 0.89 | 1.10 |
| 8631 | 0.8 | 0.90 | 1.15 |
| 8531 | 1.3 | 0.91 | 1.24 |
| 8576 | 1.5 | 0.91 | 0.93 |
| 8574 | 1.3 | 0.91 | 1.07 |
| 8687 | 1.5 | 0.92 | 1.28 |
| 8554 | 1.4 | 0.93 | 1.07 |
| 8633 | 1.2 | 0.94 | 1.27 |
| 8632 | 0.6 | 0.97 | 1.35 |
| 8684 | 1.4 | 0.98 | 0.93 |
| 8628 | 0.9 | 0.98 | 1.14 |
| 8529 | 1.2 | 0.99 | 1.04 |
| 8547 | 1.1 | 1.00 | 1.12 |
| 8647 | 0.8 | 1.02 | 0.97 |
| 8545 | 1.0 | 1.04 | 0.89 |
| 8659 | 0.8 | 1.04 | 1.17 |
| 8594 | 1.2 | 1.07 | 0.95 |
| 8655 | 1.5 | 1.08 | 1.11 |
| 8533 | 1.0 | 1.09 | 1.16 |
| 8805 | - | 1.13 | 1.27 |
| 8675 | 1.1 | 1.17 | 1.23 |
| 8579 | 1.1 | 1.18 | 1.27 |
| 8629 | 1.2 | 1.18 | 1.30 |
| 8694 | 1.4 | 1.20 | 1.14 |
| 8787 | - | 1.20 | 1.20 |
| 8688 | 1.2 | 1.20 | 1.00 |
| 8559 | 1.8 | 1.21 | 1.47 |
| 8811 | - | 1.23 | 1.50 |
| 8597 | 1.9 | 1.25 | 2.00 |

0165669

| Patient I.D. No. | $FT_4$ E.D.[a] | Single Step $FT_4$ Assay | |
| --- | --- | --- | --- |
| | | Without Salicylate[b] | With Salicylate[b] |
| 8789 | – | 1.25 | 1.41 |
| 8567 | 1.4 | 1.25 | 1.16 |
| 8556 | 1.6 | 1.25 | 1.59 |
| 8526 | 1.0 | 1.26 | 1.40 |
| 8640 | 1.9 | 1.26 | 1.32 |
| 8587 | 1.5 | 1.28 | 1.25 |
| 8541 | 1.2 | 1.28 | 1.24 |
| 8573 | 1.4 | 1.28 | 1.55 |
| 8797 | – | 1.28 | 1.41 |
| 8638 | 1.1 | 1.31 | 1.14 |
| 8534 | 1.2 | 1.32 | 1.37 |
| 8548 | 1.0 | 1.32 | 1.12 |
| 8578 | 1.1 | 1.32 | 1.57 |
| 8593 | 1.5 | 1.35 | 1.30 |
| 8537 | 1.0 | 1.36 | 1.60 |
| 8792 | – | 1.37 | 1.17 |
| 8817 | – | 1.38 | 1.43 |
| 8672 | 1.0 | 1.39 | 1.42 |
| 8615 | 1.3 | 1.39 | 1.30 |
| 8809 | – | 1.39 | 1.62 |
| 8601 | 1.3 | 1.41 | 1.32 |
| 8606 | 1.6 | 1.41 | 1.35 |
| 8701 | 1.3 | 1.41 | 1.43 |
| 8621 | 1.5 | 1.44 | 1.65 |
| 8562 | 1.3 | 1.44 | 1.40 |
| 8539 | 1.5 | 1.45 | 1.76 |
| 8641 | – | 1.46 | 1.60 |
| 8660 | 1.0 | 1.46 | 1.18 |
| 8698 | 1.4 | 1.47 | 1.41 |

| Patient I.D. No. | FT$_4$ E.D.[a] | Single Step FT$_4$ Assay | |
|---|---|---|---|
| | | Without Salicylate[b] | With Salicylate[b] |
| 8818 | - | 1.48 | 1.35 |
| 8542 | 1.2 | 1.49 | 1.40 |
| 8804 | - | 1.50 | 1.61 |
| 8549 | 1.8 | 1.51 | 1.43 |
| 8617 | 1.5 | 1.51 | 1.40 |
| 8678 | 1.1 | 1.53 | 1.40 |
| 8624 | 1.4 | 1.53 | 1.35 |
| 8551 | 1.0 | 1.53 | 1.22 |
| 8790 | - | 1.54 | 1.32 |
| 8544 | 1.5 | 1.54 | 1.47 |
| 8612 | 1.8 | 1.55 | 1.52 |
| 8801 | - | 1.59 | 1.60 |
| 8546 | 1.3 | 1.61 | 1.30 |
| 8598 | 1.6 | 1.61 | 1.33 |
| 8814 | - | 1.62 | 1.55 |
| 8692 | 1.3 | 1.62 | 1.23 |
| 8581 | 1.0 | 1.63 | 1.53 |
| 8695 | 1.8 | 1.64 | 1.85 |
| 8550 | 1.5 | 1.64 | 1.43 |
| 8530 | 1.3 | 1.64 | 1.65 |
| 8652 | 1.1 | 1.65 | 1.15 |
| 8693 | 1.0 | 1.66 | 1.40 |
| 8668 | 1.3 | 1.67 | 1.60 |
| 8540 | 1.5 | 1.67 | 1.70 |
| 8637 | 1.0 | 1.67 | 1.45 |
| 8798 | - | 1.68 | 1.50 |
| 8607 | 1.5 | 1.68 | 1.48 |
| 8596 | 1.8 | 1.69 | 1.85 |
| 8685 | 0.9 | 1.70 | 1.94 |

| Patient I.D. No. | FT$_4$ E.D.[a] | Single Step FT$_4$ Assay | |
|---|---|---|---|
| | | Without Salicylate[b] | With Salicylate[b] |
| 8527 | 1.5 | 1.70 | 1.97 |
| 8806 | – | 1.71 | 1.80 |
| 8639 | 1.3 | 1.71 | 1.45 |
| 8563 | 1.3 | 1.73 | 1.75 |
| 8603 | 1.4 | 1.73 | 1.52 |
| 8588 | 2.2 | 1.74 | 1.70 |
| 8599 | 1.7 | 1.75 | 1.37 |
| 8605 | 1.8 | 1.75 | 1.85 |
| 8800 | – | 1.75 | 1.52 |
| 8592 | 1.2 | 1.76 | 1.56 |
| 8821 | – | 1.76 | 1.50 |
| 8642 | 1.2 | 1.77 | 1.75 |
| 8600 | 1.7 | 1.77 | 1.95 |
| 8810 | – | 1.78 | 1.75 |
| 8700 | 1.1 | 1.79 | 1.40 |
| 8575 | 2.5 | 1.80 | 2.00 |
| 8611 | 1.8 | 1.81 | 1.45 |
| 8808 | – | 1.83 | 2.00 |
| 8581 | 1.8 | 1.84 | 1.58 |
| 8816 | – | 1.84 | 1.75 |
| 8815 | – | 1.85 | 1.80 |
| 8677 | 1.2 | 1.85 | 1.60 |
| 8794 | – | 1.86 | 1.63 |
| 8649 | 1.6 | 1.86 | 1.72 |
| 8791 | – | 1.87 | 1.72 |
| 8528 | 1.4 | 1.91 | 1.87 |
| 8564 | 1.5 | 1.92 | 1.80 |
| 8591 | 1.6 | 1.94 | 1.30 |
| 8555 | 1.9 | 1.95 | 2.20 |

| Patient I.D. No. | FT$_4$ E.D.[a] | Single Step FT$_4$ Assay | |
| --- | --- | --- | --- |
| | | Without Salicylate[b] | With Salicylate[b] |
| 8820 | - | 1.95 | 2.48 |
| 8819 | - | 1.96 | 1.90 |
| 8689 | 1.0 | 1.97 | 1.65 |
| 8802 | - | 1.98 | 1.78 |
| 8796 | - | 1.98 | 1.65 |
| 8699 | 1.0 | 1.99 | 1.50 |
| 8793 | - | 2.0 | 1.58 |
| 8788 | - | 2.03 | 2.00 |
| 8608 | 2.0 | 2.10 | 1.94 |
| 8602 | 1.7 | 2.12 | 2.10 |
| 8691 | 1.3 | 2.17 | 1.78 |
| 8570 | 2.0 | 2.18 | 2.53 |
| 8807 | - | 2.21 | 2.30 |
| 8795 | - | 2.25 | 2.20 |
| 8822 | - | 2.28 | 2.33 |
| 8661 | 0.9 | 2.31 | 2.05 |
| 8799 | - | 2.32 | 2.20 |
| 8785 | - | 2.33 | 2.33 |
| 8569 | 2.3 | 2.34 | 2.20 |
| 8625 | 1.5 | 2.34 | 1.85 |
| 8651 | - | 2.36 | 1.80 |
| 8803 | - | 2.36 | 1.87 |
| 8623 | 1.8 | 2.42 | 1.83 |
| 8650 | 1.6 | 2.49 | 2.40 |
| 8613 | 2.3 | 2.49 | 2.30 |
| 8679 | 1.4 | 2.64 | 2.52 |

[a] ng/dl by equilibrium dialysis

[b] ng/dl

The data presented in Table I were treated to statistical analysis in two groups: (1) samples which also were analyzed by equilibrium dialysis and (2) all samples. The statistical analyses are summarized in Table II.

## Table II

### Statistical Analysis of the Data in Table 1[a]

#### Group 1 (115 samples)[b]

|  | $FT_4$ E.D. | Single Step $FT_4$ Assay | |
|---|---|---|---|
|  |  | Without Salicylate | with Salicylate |
| Mean | 1.38 | 1.45 | 1.44 |
| ± 1SD | 0.37 | 0.47 | 0.39 |
| ± 2SD | 0.74 | 0.94 | 0.78 |

#### Group 2 (153 samples)[c]

|  | | Without Salicylate | with Salicylate |
|---|---|---|---|
| Mean | – | 1.53 | 1.50 |
| ± 1SD | – | 0.47 | 0.40 |
| ± 2SD | – | 0.94 | 0.80 |

[a]All values reported as ng/dl.

[b]Those samples which also were analyzed by equilibrium dialysis.

[c]All samples reported in Table I.

With normal individuals, the assay gave essentially the same values, on the average. Based on standard deviations, however, the use of salicylate resulted in a somewhat more precise assay.

## Example 2

The procedure of Example 1 was repeated, except that the samples employed represented abnormal patient populations. The data are summarized in Tables III through XII.

### Table III

#### Abnormal Patient Population
#### Salicylate Medication

| Sample No. | Single Step FT$_4$ Assay | | Blood Salicylate Level[b] |
| | Without Salicylate[a] | With Salicylate[a] | |
| --- | --- | --- | --- |
| 1 | 0.10 | 0.83 | 311.0 |
| 2 | 0.20 | 0.83 | 484.0 |
| 3 | 0.20 | 0.64 | 417.5 |
| 4 | 0.27 | 0.73 | 52.7 |
| 5 | 0.31 | 0.89 | 344.0 |
| 6 | 0.45 | 0.91 | 272.0 |
| 7 | 0.50 | 0.58 | 148.0 |
| 8 | 0.52 | 0.88 | 97.8 |
| 9 | 0.59 | 0.53 | 144.0 |
| 10 | 0.60 | 0.95 | 101.0 |
| 11 | 0.60 | 0.82 | 97.8 |
| 12 | 0.62 | 1.05 | 274.0 |
| 13 | 0.66 | 1.18 | 43.9 |
| 14 | 0.73 | 0.98 | 58.2 |
| 15 | 0.75 | 1.05 | 108.0 |
| 16 | 0.80 | 1.05 | 132.0 |
| 17 | 0.82 | 1.00 | <25.0 |

| | | | |
|---|---|---|---|
| 18 | 0.86 | 1.20 | 171.0 |
| 19 | 0.86 | 1.19 | 118.0 |
| 20 | 0.86 | 0.92 | 35.6 |
| 21 | 0.91 | 1.40 | 88.3 |
| 22 | 1.01 | 1.33 | <25.0 |
| 23 | 1.08 | 1.13 | 87.0 |
| 24 | 1.28 | 1.35 | 107.0 |
| 25 | 1.29 | 1.15 | <25.0 |
| 26 | 2.3 | 1.78 | <25.0 |

[a] ng/dl
[b] μg/ml

## Table IV

**Abnormal Patient Population**

**Hypothyroid**

| Patient I.D. No. | Single Step $FT_4$ Assay | |
|---|---|---|
| | Without Salicylate[a] | With Salicylate[a] |
| 8899 | 0 | 0 |
| 8905 | 0.05 | 0 |
| 9983 | 0.18 | 0.18 |
| 9986 | 0.18 | 0.18 |
| 9978 | 0.19 | 0.18 |
| 8900 | 0.20 | 0.18 |
| 9940 | 0.29 | 0.38 |
| 9991 | 0.31 | 0.25 |
| 9980 | 0.39 | 0.40 |
| 9987 | 0.46 | 0.20 |
| 9976 | 0.46 | 0.51 |

| Patient I.D. No. | Single Step FT$_4$ Assay | |
| | Without Salicylate[a] | With Salicylate[a] |
| --- | --- | --- |
| 9984 | 0.47 | 0.36 |
| 8846 | 0.47 | 0.45 |
| 8848 | 0.51 | 0.51 |
| 9982 | 0.52 | 0.47 |
| 9981 | 0.55 | 0.35 |
| 9979 | 0.59 | 0.45 |
| 8898 | 0.60 | 0.50 |
| 9992 | 0.70 | 0.69 |
| 9977 | 0.83 | 0.90 |
| 9985 | 0.97 | 0.47 |
| 9988 | 1.08 | 0.79 |

[a] ng/dl

## Table V

### Abnormal Patient Population
### Hyperthyroid

| Patient I.D. No. | Single Step FT$_4$ Assay | |
| | Without Salicylate[a] | With Salicylate[a] |
| --- | --- | --- |
| 8892 | 2.22 | 2.25 |
| 8854 | 3.72 | 3.82 |
| 8875 | 4.29 | 4.05 |
| 8894 | 4.96 | 4.3 |
| 8895 | 4.76 | 4.3 |
| 8876 | 6.32 | 6.2 |

| Patient I.D. No. | Single Step FT$_4$ Assay | |
| --- | --- | --- |
| | Without Salicylate[a] | With Salicylate[a] |
| 8850 | 6.4 | 6.4 |
| 8891 | 6.4 | 6.4 |

[a] ng/dl

## Table VI

### Abnormal Patient Population
### Sick Euthyroid

| Patient I.D. No. | Single Step FT$_4$ Assay | |
| --- | --- | --- |
| | Without Salicylate[a] | With Salicylate[a] |
| 9945 | 0.19 | 0.41 |
| 9950 | 0.62 | 0.97 |
| 9940 | 0.63 | 0.77 |
| 9942 | 0.64 | 0.74 |
| 9948 | 0.75 | 0.65 |
| 9946 | 0.81 | 0.68 |
| 9944 | 0.82 | 0.75 |
| 9949 | 1.03 | 1.00 |
| 9947 | 1.06 | 1.08 |
| 9941 | 1.07 | 1.15 |
| 9943 | 1.15 | 1.20 |

[a] ng/dl

0165669

## Table VII

Abnormal Patient Population

Dilantin Medication

| Patient I.D. No. Salicylate[a] | Single Step $FT_4$ Assay | |
|---|---|---|
| | Without Salicylate[a] | With |
| 9328 | 0.52 | 0.40 |
| 9337 | 0.59 | 0.53 |
| 9329 | 0.49 | 0.84 |
| 9326 | 0.64 | 0.92 |
| 9332 | 0.71 | 0.72 |
| 9342 | 0.78 | 0.76 |
| 9325 | 0.89 | 0.92 |
| 9334 | 0.93 | 0.54 |
| 9339 | 1.01 | 1.27 |
| 9340 | 1.08 | 1.08 |
| 9324 | 1.12 | 1.22 |
| 9338 | 1.13 | 0.86 |
| 9333 | 1.37 | 0.94 |
| 9330 | 1.44 | 1.25 |
| 9341 | 1.88 | 1.60 |
| 9336 | 2.01 | 1.17 |

[a] ng/dl

## Table VIII

### Abnormal Patient Population
### Estrogen Medication

| Patient I.D. No. | Single Step FT$_4$ Assay | |
| --- | --- | --- |
| | Without Salicylate[a] | With Salicylate[a] |
| 8851 | 0.91 | 0.89 |
| 8880 | 1.40 | 1.25 |
| 8881 | 1.60 | 1.35 |
| 8852 | 1.62 | 1.35 |
| 8885 | 1.65 | 1.40 |
| 8886 | 1.72 | 1.60 |
| 8884 | 1.75 | 1.48 |
| 8878 | 1.77 | 1.45 |
| 8856 | 1.82 | 1.74 |
| 8882 | 1.87 | 1.70 |
| 8879 | 1.92 | 1.56 |
| 8857 | 2.03 | 1.90 |
| 8855 | 2.10 | 1.83 |
| 8883 | 2.15 | 1.75 |

[a] ng/dl

## Table IX

—

### Abnormal Patient Population
### Pregnant – 1st Trimester

| Patient I.D. No. | Single Step $FT_4$ Assay | |
| --- | --- | --- |
| | Without Salicylate[a] | With Salicylate[a] |
| 9611 | 1.02 | 1.53 |
| 9603 | 1.20 | 1.14 |
| 9602 | 1.25 | 1.12 |
| 9609 | 1.56 | 1.30 |
| 9607 | 1.63 | 1.37 |
| 9617 | 1.64 | 1.63 |
| 9606 | 1.66 | 1.35 |
| 9601 | 1.68 | 1.50 |
| 9616 | 1.73 | 1.45 |
| 9615 | 1.74 | 1.64 |
| 9610 | 1.77 | 1.53 |
| 9614 | 1.82 | 1.37 |
| 9613 | 1.93 | 1.52 |
| 9600 | 1.94 | 1.63 |
| 9612 | 1.97 | 1.75 |
| 9604 | 2.02 | 1.78 |
| 9608 | 2.10 | 1.76 |

[a] ng/dl

## Table X

### Abnormal Patient Population
### Pregnant - 2nd Trimester

| Patient I.D. No. | Single Step FT$_4$ Assay | |
|---|---|---|
| | Without Salicylate[a] | With Salicylate[a] |
| 9595 | 1.18 | 1.00 |
| 9586 | 1.18 | 1.24 |
| 9591 | 1.24 | 1.23 |
| 9583 | 1.25 | 1.23 |
| 9599 | 1.28 | 1.13 |
| 9581 | 1.36 | 1.13 |
| 9597 | 1.40 | 1.25 |
| 9584 | 1.61 | 1.52 |
| 9592 | 1.65 | 1.50 |
| 9598 | 1.67 | 1.25 |
| 9587 | 1.73 | 1.60 |
| 9593 | 1.79 | 1.45 |
| 9582 | 1.88 | 1.72 |
| 9594 | 1.94 | 1.32 |
| 9589 | 1.96 | 1.93 |
| 9588 | 2.06 | 2.02 |
| 9585 | 2.15 | 2.00 |
| 9590 | 2.15 | 1.85 |
| 9596 | 2.20 | 1.17 |

[a] ng/dl

## Table XI

### Abnormal Patient Population
### Pregnant - 3rd Trimester

| Patient I.D. No. | Single Step FT$_4$ Assay | |
| --- | --- | --- |
| | Without Salicylate[a] | With Salicylate[a] |
| 9573 | 0.85 | 0.87 |
| 9571 | 0.85 | 0.96 |
| 9579 | 0.90 | 0.95 |
| 9567 | 0.98 | 1.07 |
| 9568 | 1.08 | 1.07 |
| 9570 | 1.09 | 0.98 |
| 9569 | 1.10 | 1.00 |
| 9575 | 1.30 | 1.13 |
| 9580 | 1.60 | 1.55 |
| 9596 | 1.61 | 1.68 |
| 9572 | 1.76 | 1.77 |

[a] ng/dl

# Table XII

## Abnormal Patient Population
### High TBG

| Patient I.D. No. | Single Step $FT_4$ Assay Without Salicylate[a] | With Salicylate[a] |
|---|---|---|
| 9573 | 0.85 | 0.87 |
| 9571 | 0.85 | 0.96 |
| 9579 | 0.90 | 0.95 |
| 9567 | 0.98 | 1.07 |
| 9611 | 1.02 | 1.53 |
| 9568 | 1.08 | 1.07 |
| 9586 | 1.18 | 1.24 |
| 9603 | 1.20 | 1.14 |
| 9591 | 1.24 | 1.23 |
| 9583 | 1.25 | 1.23 |
| 9599 | 1.28 | 1.13 |
| 9575 | 1.30 | 1.13 |
| 9581 | 1.36 | 1.13 |
| 9597 | 1.40 | 1.25 |
| 9580 | 1.60 | 1.55 |
| 9576 | 1.61 | 1.68 |
| 9584 | 1.61 | 1.52 |
| 9592 | 1.65 | 1.50 |
| 9598 | 1.67 | 1.25 |
| 9616 | 1.73 | 1.45 |
| 9587 | 1.73 | 1.60 |
| 9615 | 1.74 | 1.64 |
| 9572 | 1.76 | 1.77 |
| 9593 | 1.79 | 1.45 |

0165669

| Patient I.D. No. | Single Step FT$_4$ Assay | |
|---|---|---|
| | Without Salicylate[a] | With Salicylate[a] |
| 9582 | 1.88 | 1.72 |
| 9613 | 1.93 | 1.52 |
| 9594 | 1.94 | 1.32 |
| 9589 | 1.96 | 1.93 |
| 9612 | 1.97 | 1.75 |
| 9588 | 2.06 | 2.02 |
| 9590 | 2.15 | 1.85 |
| 9585 | 2.15 | 2.00 |
| 9586 | 2.20 | 1.17 |

[a] ng/dl

The effectiveness of salicylate in improving correct diagnosis for several of the abnormal patient populations summarized in Tables III through XII, inclusive, is seen from Table XIII, the most dramatic improvement occurring with respect to patients who are receiving salicylate medication.

## Table XIII

### Abnormal Patient Populations:
### Percent Diagnostically Correct
### for Given Condition

| | Single Step $FT_4$ Assay | | | |
| | Without Salicylate | | With Salicylate | |
| Condition | Number | Percent | Number | Percent |
|---|---|---|---|---|
| Salicylate Medication | 11 | 42 | 22 | 85 |
| Hypothyroid | 19 | 86 | 21 | 96 |
| Hyperthyroid | 8 | 100 | 8 | 100 |
| Sick Euthyroid | 7 | 64 | 6 | 55 |
| Dilantin Medication | 10 | 62 | 11 | 69 |
| Estrogen Medication | 14 | 100 | 14 | 100 |
| Pregnant | 47 | 100 | 47 | 100 |
| High TBG | 35 | 100 | 35 | 100 |
| Total | 151 | 84 | 164 | 92 |

## Example 3

The procedure of Example 1 was followed, except that the patient samples represented sick euthyroid patients. The data are presented in Tables XIV and XV. Table XIV summarizes patient diagnoses, while Table XV summarizes the results of the single tube free thyroxine assay without and with the addition of salicylate to tracer reagent.

### Table XIV

Sick Euthyroid
Patient Diagnosis

| Patient No. | Diagnosis |
|---|---|
| 1 | Lymphocytic Lymphoma |
| 2 | Macrocytic Anemia |
| 3 | Stroke |
| 4 | Tuberculosis, Diabetes |
| 5 | Diabetes |
| 6[a] | GI Bleeding |
| 7[a] | Soft Tissue Sarcoma |
| 8 | Renal CA, Metastatic to Brain |
| 9 | Mitral Valve Prolapse |
| 10 | Adenocarcinoma Lung, Sepsis |
| 11 | Diabetes |
| 12 | GI Bleeding, Ulcer |
| 13 | Ruptured Mitral Valve |
| 14 | Renal Failure, Atrial Fibrillation |

## Sick Euthyroid
## Patient Diagnosis

| Patient No. | Diagnosis |
|---|---|
| 15 | Bronchiectasis |
| 16 | Pneumonia, Hypertension |
| 17 | Atrial Fibrillation |
| 18 | Myocardial Infarction |
| 19 | GI Bleeding, Anemia |
| 20 | Pneumonia |
| 21 | GI Bleeding, Ulcer |
| 22 | CA Lung |
| 23 | CA Lung |
| 24 | Acute Myocardial Infarction |
| 25[a] | CA Breast |
| 26 | Myocardial Infarction |
| 27 | Heart Failure, Athrosclerosis |
| 28 | GI Bleeding |
| 29[a] | Acute Pulmonary Embolus, Rheumatoid Arthritis |
| 30 | Asthma, Chronic Lung Disease |
| 31 | Angina |
| 32 | Acute Pancreatitis |
| 33 | Acute Asthma Attack |
| 34[a] | Chest Pain |
| 35 | Lung CA |
| 36 | Acute Asthma Attack |
| 37 | Lymphoma |
| 38 | Multiple Myeloma |
| 39 | GI Bleeding, Ulcer |
| 40 | Angina, Colitis |

## Sick Euthyroid
## Patient Diagnosis

| Patient No. | Diagnosis |
|---|---|
| 41 | Acute Myocardial Infarction |
| 42 | Amnesia |
| 43 | Hypertension |
| 44 | Hyponatremia, Anemia, Diabetes |
| 45 | Acute Myocardial Infarction |
| 46 | Complete Heart Block |
| 47 | Myocardial Infarction, Basal Cell CA |
| 48[a] | Breast CA |
| 49 | Leukemia-Chronic |
| 50 | Hypertension, Anemia |
| 51 | Chronic Lung Disease |
| 52 | Systemic Lupus |
| 53 | Acute Myocardial Infarction |
| 54[a] | Acute Diabetic Ketoacidosis |
| 55 | Lymphocytic Lymphoma |
| 56 | Congestive Heart Failure |
| 57 | Lung CA |
| 58 | Cerebrovascular Accident |
| 59 | Acute Leukemia |

[a] Patient receiving thyroid medication.

## Table XV

Sick Euthyroid
Patient Results

| Patient No. | Single Step FT$_4$ Assay | |
|---|---|---|
| | Without Salicylate[a] | With Salicylate[a] |
| 1 | 1.61 | 1.59 |
| 2 | 1.09 | 1.09 |
| 3 | 1.38 | 1.24 |
| 4 | 0.84 | 0.77 |
| 5 | 1.44 | 1.35 |
| 6[b] | 0.91 | 0.90 |
| 7[b] | 1.38 | 1.21 |
| 8 | 1.61 | 1.69 |
| 9 | 0.69 | 0.86 |
| 10 | 1.15 | 1.44 |
| 11 | 1.19 | 1.19 |
| 12 | 1.63 | 1.65 |
| 13 | 1.36 | 1.57 |
| 14 | 1.65 | 1.78 |
| 15 | 1.62 | 1.38 |
| 16 | 1.74 | 1.79 |
| 17 | 1.21 | 1.37 |
| 18 | 0.21 | 0.59 |
| 19 | 1.54 | 1.53 |
| 20 | 1.33 | 1.46 |
| 21 | 1.12 | 1.17 |
| 22 | 1.72 | 1.73 |
| 23 | 1.53 | 1.60 |
| 24 | 0.55 | 0.65 |

| Patient No. | Single Step FT$_4$ Assay | |
|---|---|---|
| | Without Salicylate[a] | With Salicylate[a] |
| 25[b] | 1.33 | 1.14 |
| 26 | 0.67 | 0.73 |
| 27 | 0.01 | 0.56 |
| 28 | 1.65 | 1.66 |
| 29 | 0.75 | 0.97 |
| 30 | 1.76 | 1.72 |
| 31 | 0.94 | 1.28 |
| 32 | 0.98 | 1.13 |
| 33 | 1.37 | 1.29 |
| 34[b] | 1.69 | 1.54 |
| 35 | 1.12 | 1.12 |
| 36 | 1.40 | 1.37 |
| 37 | 1.14 | 1.08 |
| 38 | 1.45 | 1.40 |
| 39 | 1.61 | 1.51 |
| 40 | 1.30 | 1.32 |
| 41 | 1.24 | 1.27 |
| 42 | 1.49 | 1.35 |
| 43 | 1.44 | 1.45 |
| 44 | 2.25 | 2.41 |
| 45 | 2.22 | 1.91 |
| 46 | 1.02 | 1.06 |
| 47 | 1.26 | 1.17 |
| 48[b] | 1.25 | 1.26 |
| 49 | 1.48 | 1.38 |
| 50 | 1.87 | 1.90 |
| 51 | 0.31 | 0.68 |
| 52 | 2.17 | 2.73 |
| 53 | 0.64 | 1.00 |

| Patient No. | Single Step FT$_4$ Assay | |
| --- | --- | --- |
| | Without Salicylate[a] | With Salicylate[a] |
| 54[b] | 1.40 | 1.49 |
| 55 | 1.55 | 1.60 |
| 56 | 0.94 | 1.14 |
| 57 | 0.90 | 0.93 |
| 58 | 1.64 | 1.47 |
| 59 | 1.10 | 1.17 |

[a] ng/dl

[b] Patient receiving thyroid medication.

Given a normal range of 0.77-2.49 ng/dl, it is seen from Table XV that the number of patients found to be within the normal range are 51 without salicylate and 54 with salicylate. The percentage of diagnostically correct values are 86 without salicylate and 92 with salicylate.

## Example 4

The procedure of Example 1 was repeated once more, this time on patient samples taken before and after dialysis. The data are summarized in Tables XVI and XVIII.

## Table XVI

## Pre-Dialysis Results

| Patient No. | I.D. No. | Single Step $FT_4$ Assay | |
|---|---|---|---|
| | | Without Salicylate[a] | With Salicylate[a] |
| 1 | 8282 | 1.18 | 1.55 |
| 2 | 8286 | 0.93 | 1.27 |
| 3 | 8288 | 0.0 | 0.40 |
| 4 | 8290 | 1.16 | 1.20 |
| 5 | 8292 | 1.32 | 1.75 |
| 6 | 8294 | 0.73 | 0.96 |
| 7 | 8296 | 1.28 | 1.75 |
| 8 | 8298 | 1.11 | 1.12 |

[a]ng/dl

The number of patients within the normal range when assayed without and with salicylate are 6 and 7, respectively, while the percent diagnostically correct are 75 and 87.5, respectively.

## Table XVII

## Post-Dialysis Results

| | | Single Step $FT_4$ Assay | |
|---|---|---|---|
| Patient No. | I.D. No. | Without Salicylate | With Salicylate |
| 1 | 8283 | 0.61 | 0.87 |
| 2 | 8287 | 0.23 | 0.96 |
| 3 | 8289 | 0.13 | 0.48 |
| 4 | 8291 | 0.94 | 1.05 |
| 5 | 8293 | 0.09 | 0.77 |
| 6 | 8295 | 0.54 | 1.27 |
| 7 | 8297 | 0.32 | 0.86 |
| 8 | 8299 | 0.0 | 0.59 |

[a] ng/dl

The number of patients within the normal range when assayed without and with salicylate are 1 and 6, respectively, a significant improvement resulting from the use of salicylate. The percent diagnostically correct without and with salicylate are 12.5 and 75, respectively.

0165669

As already indicated, it is to be understood that the foregoing detailed description is given merely by way of illustration and that many variations may be made therein without departing from the spirit and scope of the invention.

CLAIMS:

1.         Method of determining the concentration of a free ligand in a liquid sample which contains the ligand in both free and bound states, which method comprises the steps of:

A.   adding to the liquid sample labeled ligand or labeled ligand derivative which does not bind significantly to the specific endogenous ligand binding protein, but does bind significantly to other proteins, and a binder specific for the ligand and labeled ligand or labeled ligand derivative;

B.   incubating the mixture resulting from step A;

C.   measuring the amount of labeled ligand or labeled ligand derivative which is bound to the specific binder; and

D.   using the measurement to determine the concentration of the free ligand in the liquid sample; the improvement which comprises adding to the sample in step A an amount of a blocking agent which is sufficient to prevent significant binding of the labeled ligand or labeled ligand derivative to endogenous and/or exogenous ligand binding proteins without causing significant release of bound ligand.

2.   Method of determining the concentration of a free ligand in a liquid sample which contains the ligand in both free and bound states, which method comprises the steps of:

A.    adding to the liquid sample labeled ligand or labeled ligand derivative which does not bind significantly to the specific endogenous ligand binding protein, but does bind significantly to other proteins, and a binder specific for ligand and labeled ligand or labeled ligand derivative, which binder is immobilized on a water-insoluble support;

B.    incubating the mixture resulting from step A;

C.    separating the incubated mixture into a solid phase and a liquid phase, the solid phase comprising immobilized specific binder having bound thereto ligand and labeled ligand or labeled ligand derivative;

D.    measuring the amount of labeled ligand or labeled ligand derivative present in either the solid or liquid phase; and

E.    comparing the measurement obtained in step D with a dose-response curve prepared from samples of standard solutions containing known concentrations of free ligand in order to determine the concentration of the free ligand in the liquid sample;

the improvement which comprises adding to the sample in step A an amount of a blocking agent which is sufficient to prevent significant binding of the labeled ligand or labeled ligand derivative to endogenous and/or exogenous ligand binding proteins without causing significant release of bound ligand.

3.    Method according to claims 1 or 2, characterized in that the specific binder is immobilized on a water-insoluble support, or on a water-insoluble particulate support, or on a water-insoluble particulate paramagnetic support.

4.    Method according to claim 3, characterized in that the specific binder is antiligand antibody.

5.    Method according to any one of claims 1-4, characterized in that the ligand is a thyroid hormone.

6.    Method according to claim 5, characterized in that the ligand is thyroxine or 3,5,3'-triiodothyronine.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 015 687 (R.P. EKINS) * Whole document * | 1-6 | G 01 N 33/53 G 01 N 33/543 G 01 N 33/546 G 01 N 33/551 G 01 N 33/78 |
| X | EP-A-0 078 477 (MILES LABORATORIES INC.) * Page 8, lines 9-28 * | 1-6 | |
| P,X | EP-A-0 133 464 (MILES LABORATORIES INC.) * Page 3, line 19 - page 6, line 25 * | 1-6 | |
| D,X | US-A-3 911 096 (I.J. CHOPRA) * Whole document * | 1-6 | |
| A | EP-A-0 089 806 (AMERSHAM INTERNATIONAL PLC) * Whole document * | 1-6 | **TECHNICAL FIELDS SEARCHED (Int. Cl.4)** |
| A | WO-A-8 303 306 (R.P. EKINS) * Whole document * | 1-6 | G 01 N |
| A | WO-A-8 401 031 (R.P. EKINS) * Whole document * | 1-6 | |

The present search report has been drawn up for all claims

| Place of search THE HAGUE | Date of completion of the search 14-08-1985 | Examiner GRIFFITH G. |
|---|---|---|